(19) Europäisches Patentamt
European Patent Office
Office européen des brevets

(11) Publication number: **0 440 688 B1**

# EUROPEAN PATENT SPECIFICATION

(45) Date of publication of patent specification: **21.09.94**  (51) Int. Cl.⁵: **A61K  37/02**, C07K 7/10

(21) Application number: **89911721.2**

(22) Date of filing: **16.10.89**

(86) International application number:
**PCT/US89/04625**

(87) International publication number:
**WO 90/04407 (03.05.90 90/10)**

(54) **CPF PEPTIDE COMPOSITIONS AND USES.**

(30) Priority: **21.10.88 US 260861**
**07.06.89 US 362689**

(43) Date of publication of application:
**14.08.91 Bulletin  91/33**

(45) Publication of the grant of the patent:
**21.09.94 Bulletin  94/38**

(84) Designated Contracting States:
**AT BE CH DE FR GB IT LI LU NL SE**

(56) References cited:
**US-A- 4 507 230**

**THE JOURNAL OF BIOLOGICAL CHEMISTRY,
vol. 261, no. 8, 15th March 1986,
pages 3676-3680, Baltimore, US; K. RICHTER
et al.: "Sequence of preprocaerulein
cDNA cloned from skin of Xenopus laevis"**

(73) Proprietor: **THE CHILDREN'S HOSPITAL OF
PHILADELPHIA
34th Street & Civic Center Boulevard
Philadelphia, PA 19104 (US)**

(72) Inventor: **ZASLOFF, Michael
274 Linden Lane
Merion Station, PA 19066 (US)**

(74) Representative: **LOUIS, PÖHLAU, LOHRENTZ &
SEGETH
Postfach 3055
D-90014 Nürnberg (DE)**

THE JOURNAL OF BIOLOGICAL CHEMISTRY, vol. 261, no. 12, 25th April 1986, pages5341-5349, Baltimore, US; B.W. GIBSON et al.: "Novel peptide fragmentsoriginating from PGLa and the Caerulein and Xenopsin precursors from XenopusLaevis"

NUCLEIC ACID RESEARCH, vol. 13, no. 6, 1985, pages 1817-1828; T. WAKABAYASHI etal.: "Complete nucleotide sequence of mRNA for caerulein precursor from Xenopusskin: the mRNA contains an unusual repetitive structure"

**EP 0 440 688 B1**

**Description**

The present invention relates to pharmaceutical uses and compositions of certain peptides known as CPF peptides (CPF = caerulein precursor fragment) and analogues and derivatives thereof.

Some of the CPF peptides which are used in the present invention have been described in the literature and comprise the following sequences:

(1) GFGSFLGLALKAALKIGANALGGAPQQ
(2) GLASFLGKALKAGLKIGAHLLGGAPQQ
(3) GLASLLGKALKAGLKIGTHFLGGAPQQ
(4) GLASLLGKALKATLKIGTHFLGGAPQQ
(5) GFASFLGKALKAALKIGANMLGGTPQQ
(6) GFGSFLGKALKAALKIGANALGGAPQQ
(7) GFGSFLGKALKAALKIGANALGGSPQQ
(8) GFASFLGKALKAALKIGANLLGGTPQQ

The above is expressed as single letter code for amino acids.

A review of the CPF peptides can be found in Richter K. Egger, R., and Kreil (1986) J. Biol. Chem 261, 3676-3680; Wakabayashi, T., Kato, H., and Tachibaba, S. (1985) Nucleic Acids Research 13, 1817-1828; Gibson, B.W., Poulter, L., Williams, D.H., and Maggio, J.E. (1986) J. Biol. Chem 261, 5341-5349.

It should be noted that no function has been ascribed to these peptides in prior published reports. Indeed, Kreil et al state in a recent paper: "At present it is not known whether these peptides have any biological activity." (Vlasak, R., Wiborg, O., Richter, K., Burgschwaiger, J.V., and Kreil, G., (1987) Eur. J. Biochem. 169, p. 53).

In accordance with one aspect of the present invention, there is provided a pharmaceutical composition comprising at least one peptide which is a CPF peptide or a biologically active CPF analogue or derivative thereof including the following basic structure X:

$-R_1-R_1-R_2-R_2-R_1-R_1-R_3-R_1-R_1-R_1-R_3-R_1-R_1-R_4-R_5-R_1-$

wherein $R_1$ is a hydrophobic amino acid, $R_2$ is a basic hydrophillic amino acid or a hydrophobic amino acid, $R_3$ is a basic hydrophilic amino acid, $R_4$ is a hydrophobic or neutral hydrophilic amino acid, and $R_5$ is a basic or neutral hydrophilic amino acid;

or the structure: Y-X-,

wherein X is the basic structure defined above and Y is $R_5$-, $R_2-R_5$-, $R_1-R_2-R_5$- or $R_2-R_1-R_2-R_5$, and preferably Glycine-$R_1-R_2-R_5$;

or the structure: X-Z-,

wherein X is the basic structure defined above and Z is $R_1$, $R_1-R_1$, $R_1-R_1-R_4$, $R_1-R_1-R_4-R_4$, $R_1-R_1-R_4-R_4-R_6$, $R_1-R_1-R_4-R_4-R_6$-Gln, or $R_1-R_1-R_4-R_4-R_6$-Gln-Gln, wherein $R_6$ is proline or a hydrophobic amino acid;

or the structure: $(Y)_a - X - (Z)_b$

wherein Y and Z are as previously defined and a is 0 or 1 and b is 0 or 1, and a pharmaceutical carrier.

In accordance with another aspect of the invention there is provided a use of a CPF peptide or CPF analogue or derivative thereof as a ionophore or an ion channel forming peptide or for increasing the permeability of ions across a biological membrane, in particular as a substance with such properties for the manufacture of a medicament or pharmaceutical composition Accordingly, there is provided a process wherein a CPF peptide and/or biologically active derivative thereof is administered to inhibit growth of a target cell or cells.

The term derivative encompasses herein a peptide, which is a CPF peptide or biologically active analogue thereof, including additional amino acids at the amino or carboxyl end or both the amino and carboxyl end, in general the derivative does not include more than 40 amino acids.

CPF peptides as well as analogues and derivatives thereof are herein sometimes referred to collectively as CPF peptides.

The CPF peptide is preferably one which includes the following basic peptide structure x:

$-R_1-R_1-R_2-R_2-R_1-R_1-R_3-R_1-R_1-R_1-R_3-R_1-R_1-R_4-R_5-R_1-$

wherein $R_1$ is a hydrophobic amino acid;

$R_2$ in a hydrophobic amino acid or a basic hydrophilic amino acid;

$R_3$ is a basic hydrophilic amino acid;

$R_4$ is a hydrophobic or neutral hydrophilic amino acid; and

$R_5$ is a basic or neutral hydrophilic amino acid.

The hereinabove basic structure is hereinafter symbolically indicated as X.

The hydrophobic amino acids are Ala, Cys, Phe, Gly, Ile, Leu, Met, Val, Trp, and Tyr.

The neutral hydrophilic amino acids are Asn, Gln, Ser, and Thr.

3

The basic hydrophilic amino acids are Lys, Arg, and His.

The CPF peptide may include only the hereinabove noted amino acids or may include additional amino acids at the amino and/or carboxyl end or both the amino and carboxyl end. In general, the peptide does not include more than 40 amino acids.

The CPF peptides including the above basic structure preferably have from 1 to 4 additional amino acids at the amino end.

Accordingly, such preferred peptides may be represented by the structural formula:

Y - X -

wherein X is the hereinabove described basic peptide structure and Y is

(i) $R_5$-, or

(ii) $R_2$-$R_5$-; or

(iii) $R_1$-$R_2$-$R_5$; or

(iv) $R_2$-$R_1$-$R_2$-$R_5$; preferably Glycine - $R_1$-$R_2$-$R_5$.

wherein $R_1$, $R_2$ and $R_5$ are as previously defined.

The carboxyl end of the basic peptide structure may also have additional amino acids which may range from 1 to 13 additional amino acids.

In a preferred embodiment, the basic structure may have from 1 to 7 additional amino acids at the carboxyl end, which may be represented as follows:

-X - Z wherein

X is the hereinabove defined basic peptide structure and Z is

(i) $R_1$-, or

(ii) $R_1$-$R_1$-; or

(iii) $R_1$-$R_1$-$R_4$, or

(iv) $R_1$-$R_1$-$R_4$-$R_4$; or

(v) $R_1$-$R_1$-$R_4$-$R_4$-$R_6$; or

(vi) $R_1$-$R_1$-$R_4$-$R_4$-$R_6$-Gln; or

(vii) $R_1$-$R_1$-$R_4$-$R_4$-$R_6$-Gln-Gln, wherein $R_1$ and $R_4$ are as previously defined, and $R_6$ is proline or a hydrophobic amino acid.

Preferred peptides may be represented by the following structural formula

$(Y)_a$ - X - $(Z)_b$

wherein X, Y and Z are as previously defined and a is 0 or 1 and b is 0 or 1.

As representative examples of CPF peptides used in the present invention, there may be mentioned peptides represented by the following (single letter amino acid code):

G12S3LG4ALKA5LKIG678LGG9(10)QQ

Where:

1 = F, L

2 = G, A

3 = F, L

4 = K, L

5 = A, G, T

6 = A, T

7 = H, N

8 = A, M, F, L

9 = A, S, T

10 = P, L

The numbered amino acids may be employed as described in any combination to provide either a basic CPF peptide structure or an analogue or derivative thereof.

The CPF peptides and/or analogues and/or derivatives thereof are ion channel forming peptides. An ion channel forming peptide or ionophore is one which increases the permeability for ions across a natural or synthetic lipid membrane. Christensen et al. PNAS Vol. 85 P. 5072-76 (July 1988) describes methodology which indicates whether or not a peptide has ion channel properties and is therefore an ionophore. A used herein an ion channel-forming peptide is a peptide which has ion channel-forming properties as determined by the method of Christensen, et al.

In general, the CPF peptides and/or analogues or derivatives thereof are generally water soluble to a concentration of at least 20 mg/ml at neutral pH in water. In addition, such peptides are non-hemolytic; i.e., they will not rupture blood cells at effective concentrations. In addition, the structure of such peptide provides for flexibility of the peptide molecule. When the peptide is placed in water, it does not assume an amphiphilic structure. When the peptide encounters an oily surface or membrane, the peptide chain folds upon itself into a rod-like structure.

The CPF peptides and/or analogues or derivatives thereof may be administered to a host; for example a human or non-human animal, in an amount effective to inhibit growth of a target cell. Thus, for example, the CPF peptides and/or analogues or derivatives thereof may be used as antimicrobial agents anti-viral agents, antibiotics, anti-tumor agents, spermicides, as well as exhibiting other bioactive functions.

The term "antimicrobial" as used herein means that the polypeptides of in the present invention inhibit, prevent, or destroy the growth or proliferation of microbes such as bacteria, fungi, viruses, or the like.

The term "antibiotic" as used herein means that the polypeptides employed in the present invention produce effects adverse to the normal biological functions of the cell, tissue, or organism including death or destruction and prevention of the growth or proliferation of the biological system when contacted with the polypeptides.

The term "spermicidal" as used herein means that the polypeptides employed in the present invention, inhibit, prevent, or destroy the motility of sperm.

The term "antiviral" as used herein means that the polypeptides employed in the present invention inhibit, prevent, or destroy the growth or proliferation of viruses.

The term anti-tumor as used herein means that the polypeptide inhibits the growth of or destroys tumors.

The polypeptides of the present invention have a broad range of potent antibiotic activity against a plurality of microorganisms including gram-positive and gram-negative bacteria, fungi, protozoa, and the like. The polypeptides of the present invention allow a method for treating or controlling microbial infection caused by organisms which are sensitive to the polypeptides. Such treatment may comprise administering to a host organism or tissue susceptible to or affiliated with a microbial infection an antimicrobial amount of at least one of the polypeptides.

Because of the antibiotic properties of the polypeptides, they may also be used as preservatives or sterilants of materials susceptible to microbial contamination.

The CPF peptide and/or derivatives or analogues thereof may be administered in combination with a non-toxic pharmaceutical carrier or vehicle such as a filler, non-toxic buffer, or physiological saline solution. Such pharmaceutical compositions may be used topically or systemically and may be in any suitable form such as a liquid, solid, semi-solid, injectable solution, tablet, ointment, lotion, paste, capsule, or the like. The polypeptide compositions may also be used in combination with adjuvants, protease inhibitors, or compatible drugs where such a combination is seen to be desirable or advantageous in controlling infection caused by harmful microorganisms including protozoa viruses, and the like.

The peptide(s) of the present invention may be administered to a host; in particular an animal, in an effective antibiotic and/or anti-tumor and/or anti-viral and/or anti-microbial and/or an antispermicidal amount.

Depending on the use, a composition in accordance with the invention will contain an effective anti-microbial amount and/or an effective antispermicidal amount and/or an effective anti-viral amount and/or an effective anti-tumor amount and/or an effective anti-biotic amount of one or more of the hereinabove described peptides which have such activity.

The peptides, when used in topical compositions, are generally present in an amount of at least 0.1%, by weight. In most cases, it is not necessary to employ the peptide in an amount greater than 1.0%, by weight.

In employing such compositions systemically (intramuscular, intravenous, intraperitoneal), the active peptide is present in an amount to achieve a serum level of the peptide of at least about 5 ug/ml. In general, the serum level of peptide need not exceed 500 ug/ml. A preferred serum level is about 100 ug/ml. Such serum levels may be achieved by incorporating the peptide in a composition to be administered systemically at a dose of from 1 to about 10 mg/kg. In general, the peptide(s) need not be administered at a dose exceeding 100 mg/kg.

The present invention will be further described with respect to the following examples; however, the scope of the invention is not to be limited thereby:

TABLE I demonstrates the antibacterial activity of the peptide GFGSFLGLALKAALKIGANALGGAPQQ [CPF (I)] and the peptide GFASFLGKALKAALKIGANLLGGTPQQ [CPF (II)].

TABLE II shows the minimal inhibitory concentrations of CPF (I) and CPF (II) against several strains of bacteria and fungi.

5

TABLE III lists the minimal effective concentrations of CPF (I) and CPF (II) necessary to physically disrupt several different species of protozoa. In each case the peptide induces osmotic swelling.

TABLE I

| Antibacterial Activity of Peptides | |
|---|---|
| Peptide | Zone of Inhibition on Lawn of E. coli (50 $\mu$g of peptide) (mm) |
| CPF (I) | 15 |
| CPF (II) | 15 |

# TABLE II

## SPECTRUM OF ANTI-MICROBIAL ACTIVITY
## OF SYNTHETIC PEPTIDES

### ORGANISM  MINIMAL INHIBITORY CONCENTRATION ($\mu$g/ml)

| | CPF (I) | CPF (II) |
|---|---|---|
| E. coli | 10-50 | 10-50 |
| P. aeruginosa | 50-100 | 50-100 |
| S. pyogenes | 100-200 | 100-200 |
| S. cervisiae | 100-200 | 100-200 |
| C. albicans | 50-100 | 50-100 |

TABLE III

| SENSITIVITY OF PROTOZOA TO SYNTHETIC PEPTIDES Minimal Disruptive Concentration ($\mu$g/ml) | | |
|---|---|---|
| | CPF (I) | CPF (II) |
| P. caudatum | 10 | 10 |
| T. pyriformis | 10 | 10 |
| A. castellani | 2 | 2 |

TABLE IV

| ACTIVITY AGAINST MALIGNANT CELLS ($\mu$g/ml) | | |
|---|---|---|
| | CPF (I) | CPF (II) |
| Vero cells | 250 | 250 |
| Ehrlich Ascites | 250 | 250 |

(Legend) Cells were grown in MEM with 10% fetal calf serum. Peptide was added directly to a well containing a confluent lawn of cells and incubated at 37ºC for 30 min. The concentration at which greater than 90% of the cells were killed as determined by permeabilization to Trypan Blue is noted.

**Claims**

1.  A pharmaceutical composition comprising at least one peptide which is a CPF peptide or a biologically active analogue thereof including the following basic structure X:

    $-R_1-R_1-R_2-R_2-R_1-R_1-R_3-R_1-R_1-R_1-R_3-R_1-R_1-R_4-R_5-R_1-$

    wherein $R_1$ is a hydrophobic amino acid, $R_2$ is a basic hydrophilic amino acid or a hydrophobic amino acid, $R_3$ is a basic hydrophilic amino acid, $R_4$ is a hydrophobic or neutral hydrophilic amino acid, and $R_5$ is a basic or neutral hydrophilic amino acid;

    or the structure: Y-X-,

    wherein X is the basic structure defined above and Y is $R_5-$, $R_2-R_5-$, $R_1-R_2-R_5-$ or $R_2-R_1-R_2-R_5$, and preferably Glycine-$R_1-R_2-R_5$;

    or the structure: X-Z-,

    wherein X is the basic structure defined above and Z is $R_1$, $R_1-R_1$, $R_1-R_1-R_4$, $R_1-R_1-R_4-R_4$, $R_1-R_1-R_4-R_4-R_6$, $R_1-R_1-R_4-R_4-R_6$-Gln, or $R_1-R_1-R_4-R_4-R_6$-Gln-Gln, wherein $R_6$ is proline or a hydrophobic amino acid;

    or the structure: $(Y)_a - X - (Z)_b$

    wherein Y and Z are as previously defined and a is 0 or 1 and b is 0 or 1, and a pharmaceutical carrier.

2.  A pharmaceutical composition comprising at least one derivative, which is a CPF peptide or biologically active analogue thereof as claimed in Claim 1, including additional amino acids at the amino or carboxyl end or both the amino and carboxyl end, in general the derivative including not more than 40 amino acids, and a pharmaceutical carrier.

3.  A pharmaceutical composition as claimed in Claim 1 or Claim 2, wherein the CPF peptide has the following structure:

    G12S3LG4ALKA5LKIG678LGG9(10)QQ

    wherein: 1 = F, L; 2 = G, A; 3 = F, L; 4 = K, L; 5 = A, G, T; 6 = A, T; 7 = H, N; 8 = A, M, F, L; 9 = A, S, T; 10 = P, L.

4.  A pharmaceutical composition as claimed in Claim 1 or Claim 2, wherein the CPF peptide is including anyone of the following structures:-
    (1) GFGSFLGLALKAALKIGANALGGAPQQ
    (2) GLASFLGKALKAGLKIGAHLLGGAPQQ
    (3) GLASLLGKALKAGLKIGTHFLGGAPQQ
    (4) GLASLLGKALKATLKIGTHFLGGAPQQ
    (5) GFASFLGKALKAALKIGANMLGGTPQQ
    (6) GFGSFLGKALKAALKIGANALGGAPQQ
    (7) GFGSFLGKALKAALKIGANALGGSPQQ
    (8) GFASFLGKALKAALKIGANLLGGTPQQ

5.  The composition as claimed in Claims 1 to 4 wherein said CPF peptide or biologically active CPF analogue or derivative thereof is present in an amount effective to inhibit growth of a target cell.

6.  Use of a CPF peptide or CPF analogue or derivative thereof as claimed in Claims 1 to 4 as a ionophore or an ion channel forming peptide or for increasing the permeability of ions across a biological membrane, in particular as a substance with such properties for the manufacture of a medicament or composition.

7.  Use of a CPF peptide or CPF analogue or derivative thereof as claimed in Claims 1 to 4 for the manufacture of a medicament or composition effective to inhibit growth of a target cell.

8.  Use of a CPF peptide or CPF analogue or derivative thereof as claimed in Claims 1 to 4 for the manufacture of a medicament or composition, being an anti-tumour agent.

9.  Use of a CPF peptide or CPF analogue or derivative thereof as claimed in Claims 1 to 4 for the manufacture of a medicament or composition, being an anti-viral agent.

**10.** Use of a CPF peptide or CPF analogue or derivative thereof as claimed in Claims 1 to 4 for the manufacture of a medicament, being an anti-microbial agent.

**11.** Use of a CPF peptide or CPF analogue or derivative thereof as claimed in Claims 1 to 4 for the manufacture of a medicament or composition, being an anti-bacterial agent.

**12.** Use of a CPF peptide or CPF analogue or derivative thereof as claimed in Claims 1 to 4 for the manufacture of a medicament or composition, being an anti-spermicidal agent.

**13.** Use of a CPF peptide or CPF analogue or derivative thereof as claimed in Claims 1 to 4 for the manufacture of medicament or composition, being an agent against protozoes.

**14.** Use of a CPF peptide or CPF analogue or derivative thereof as claimed in Claims 1 to 4 for the manufacture of a medicament or composition, being an antibiotic.

**Patentansprüche**

**1.** Pharmazeutische Zusammensetzung umfassend mindestens ein Peptid, das ein CPF Peptid oder ein biologisch aktives Analoges davon ist, und die folgende Basisstruktur X enthält:

$-R_1-R_1-R_2-R_2-R_1-R_1-R_3-R_1-R_1-R_1-R_3-R_1-R_1-R_4-R_5-R_1-$

worin R1 eine hydrophobe Aminosäure ist, R2 eine basische hydrophile Aminosäure oder eine hydrophobe Aminosäure, R3 eine basische hydrophile Aminosäure, R4 eine hydrophobe oder neutrale hydrophile Aminosäure, und R5 eine basische oder neutrale hydrophile Aminosäure ist;

oder die Struktur: Y-X-,

worin X die vorstehend definierte Basisstruktur bedeutet und X R5-, R2-R5-, R1-R2-R5, oder R2-R1-R2-R5 bedeutet, und vorzugsweise Glycin-R1-R2-R5;

oder die Struktur: X-Z-,

worin X die vorstehend definierte Basisstruktur bedeutet und Z R1, R1-R1, R1-R1-R4, R1-R1-R4-R4, R1-R1-R4-R4-R6, R1-R1-R4-R4-R6-Gln, oder R1-R1-R4-R4-R6-Gln-Gln ist, worin R6 Prolin oder eine hydrophobe Aminosäure ist;

oder die Struktur: (Y)a - X - (Z)b

worin Y und Z die vorstehend angegebene Bedeutung besitzen und a 0 oder 1 ist, und b 0 oder 1 ist, und einen pharmazeutischen Träger.

**2.** Pharmazeutische Zusammensetzung, umfassend mindestens ein Derivat, das ein CPF Peptid oder ein biologisch aktives Analoges davon ist, gemäß Anspruch 1, dadurch gekennzeichnet, daß es zusätzliche Aminosäuren am Amino- oder Carboxylende oder am Amino- und Carboxylende enthält, wobei das Derivat im allgemeinen nicht mehr als 40 Aminosäuren enthält, und einen pharmazeutischen Träger.

**3.** Pharmazeutische Zusammensetzung nach Anspruch 1 oder Anspruch 2, dadurch gekennzeichnet, daß das CPF Peptid die folgende Struktur besitzt:

G12S3LG4ALKA5LKIG678LGG9(10)QQ

worin: 1 = F, L; 2 = G, A; 3 = F, L; 4 = K, L; 5 = A, G, T; 6 = A, T; 7 = H, N; 8 = A, M, F, L; 9 = A, S, T; 10 = P, L.

**4.** Pharmazeutische Zusammensetzung nach Anspruch 1 oder 2, dadurch gekennzeichnet, daß das CPF Peptid eine der folgenden Strukturen enthält:

(1) GFGSFLGLALKAALKIGANALGGAPQQ
(2) GLASFLGKALKAGLKIGAHLLGGAPQQ
(3) GLASLLGKALKAGLKIGTHFLGGAPQQ
(4) GLASLLGKALKATLKIGTHFLGGAPQQ
(5) GFASFLGKALKAALKIGANMLGGTPQQ
(6) GFGSFLGKALKAALKIGANALGGAPQQ
(7) GFGSFLGKALKAALKIGANALGGSPQQ
(8) GFASFLGKALKAALKIGANLLGGTPQQ

**5.** Zusammensetzung nach einem der Ansprüche 1 bis 4, dadurch gekennzeichnet, daß das CPF-Peptid oder biologisch aktive CPF-Analoge oder Derivat davon in einer Menge vorhanden ist, die wirksam ist

EP 0 440 688 B1

zur Inhibierung des Wachstums einer Empfängerzelle.

6. Verwendung eines CPF-Peptids oder CPF-Analogen oder Derivates davon gemäß einem der Ansprüche 1 bis 4 als Ionophor oder Ionenkanal-bildendes Peptid oder zur Erhöhung der Permeabilität von Ionen durch eine biologische Membran, insbesondere als Substanz mit solchen Eigenschaften zur Herstellung eines Arzneimittels oder einer Arzneimittelzusammensetzung.

7. Verwendung eines CPF-Peptids oder CPF-Analogen oder Derivates davon gemäß einem der Ansprüche 1 bis 4 zur Herstellung eines Arzneimittels oder einer Zusammensetzung mit einer das Wachstum von Empfängerzellen inhibierenden Wirksamkeit.

8. Verwendung eines CPF-Peptids oder CPF-Analogen oder Derivates davon gemäß einem der Ansprüche 1 bis 4 zur Herstellung eines Arzneimittels oder einer Zusammensetzung, die ein Antitumormittel ist.

9. Verwendung eines CPF-Peptids oder CPF-Analogen oder Derivates davon nach einem der Ansprüche 1 bis 4 zur Herstellung eines Arzneimittels oder einer Zusammensetzung, das/die ein antivirales Mittel ist.

10. Verwendung eines CPF-Peptids oder CPF-Analogen oder Derivates davon gemäß einem der Ansprüche 1 bis 4 zur Herstellung eines Arzneimittels das ein antimicrobielles Mittel ist.

11. Verwendung eines CPF-Peptids oder CPF-Analogen oder Derivates davon gemäß einem der Ansprüche 1 bis 4 zur Herstellung eines Arzneimittels oder einer Zusammensetzung, das/die ein antibakterielles Mittel ist.

12. Verwendung eines CPF-Peptids oder CPF-Analogen oder Derivates davon nach einem der Ansprüche 1 bis 4 zur Herstellung eines Arzneimittels oder einer Zusammensetzung, das/die ein antispermizides Mittel ist.

13. Verwendung eines CPF-Peptids oder CPF-Analogen oder Derivates davon gemäß einem der Ansprüche 1 bis 4 zur Herstellung eines Arzneimittels oder einer Zusammensetzung, das/die ein Mittel gegen Protozoen ist.

14. Verwendung eines CPF-Peptids oder CPF-Analogen oder Derivates davon nach einem der Ansprüche 1 bis 4 zur Herstellung eines Arzneimittels oder einer Zusammensetzung, das/die ein Antibiotikum ist.

**Revendications**

1. Composition pharmaceutique comprenant au moins un peptide qui est un peptide CPF ou un analogue biologiquement actif de celui-ci comprenant la structure de base X suivante :
$$-R_1-R_1-R_2-R_2-R_1-R_1-R_3-R_1-R_1-R_1-R_3-R_1-R_1-R_4-R_5-R_1-$$
dans laquelle $R_1$ représente un aminoacide hydrophobe, $R_2$ représente un aminoacide hydrophile basique ou un aminoacide hydrophobe, $R_3$ représente un aminoacide hydrophile basique, $R_4$ représente un aminoacide hydrophobe ou hydrophile neutre et $R_5$ représente un aminoacide hydrophile basique ou neutre;
ou la structure : Y-X-,
dans laquelle X représente la structure de base définie ci-dessus et Y représente $R_5$-, $R_2$-$R_5$-, $R_1$-$R_2$-$R_5$- ou $R_2$-$R_1$-$R_2$-$R_5$-, et avantageusement glycine-$R_1$-$R_2$-$R_5$,
ou la structure : X-Z-,
dans laquelle X représente la structure de base définie ci-dessus et Z représente $R_1$, $R_1$-$R_1$, $R_1$-$R_1$-$R_4$, $R_1$-$R_1$-$R_4$-$R_4$, $R_1$-$R_1$-$R_4$-$R_4$-$R_6$, $R_1$-$R_1$-$R_4$-$R_4$-$R_6$-Gln ou $R_1$-$R_1$-$R_4$-$R_4$-$R_6$-Gln-Gln, où $R_6$ représente de la proline ou un aminoacide hydrophobe,
ou la structure : $(Y)_a$ - X - $(Z)_b$,
dans laquelle Y et Z sont tels que définis précédemment et a est égal à 0 ou 1 et b est égal à 0 ou 1, et un support pharmaceutique.

2. Composition pharmaceutique comprenant au moins un dérivé, qui est un peptide CPF ou un analogue biologiquement actif de celui-ci suivant la revendication 1, comprenant des aminoacides additionnels à

9

l'extrémité amino ou carboxyle ou aux extrémités amino et carboxyle, le dérivé ne comprenant pas plus d'une manière générale de 40 aminoacides, et un support pharmaceutique.

3. Composition pharmaceutique suivant l'une ou l'autre des revendications 1 et 2, dans lequel le peptide CPF a la structure suivante :

G12S3LG4ALKA5LKIG678LGG9(10)QQ

où : 1 = F, L; 2 = G, A; 3 = F, L; 4 = K, L; 5 = A, G, T; 6 = A, T; 7 = H, N; 8 = A, M, F, L; 9 = A, S, T; 10 = P, L.

4. Composition pharmaceutique suivant l'une ou l'autre des revendications 1 et 2, dans laquelle le peptide CPF comprend l'une quelconque des structures suivantes :
(1) GFGSFLGLALKAALKIGANALGGAPQQ
(2) GLASFLGKALKAGLKIGAHLLGGAPQQ
(3) GLASLLGKALKAGLKIGTHFLGGAPQQ
(4) GLASLLGKALKATLKIGTHFLGGAPQQ
(5) GFASFLGKALKAALKIGANMLGGTPQQ
(6) GFGSFLGKALKAALKIGANALGGAPQQ
(7) GFGSFLGKALKAALKIGANALGGSPQQ
(8) GFASFLGKALKAALKIGANLLGGTPQQ

5. Composition suivant l'une quelconque des revendications 1 à 4, dans laquelle le peptide CPF ou son analogue ou dérivé CPF biologiquement actif est présent en une quantité efficace pour inhiber la croissance d'une cellule cible.

6. Utilisation d'un peptide CPF ou d'un analogue ou dérivé CPF de celui-ci suivant l'une quelconque des revendications 1 à 4, comme ionophore ou peptide formateur de canaux ioniques ou pour augmenter la perméabilité d'ions à travers une membrane biologique, en particulier comme substance avec de telles propriétés pour la fabrication d'un médicament ou d'une composition.

7. Utilisation d'un peptide CPF ou d'un analogue ou dérivé CPF de celui-ci suivant l'une quelconque des revendications 1 à 4, pour la fabrication d'un médicament ou d'une composition efficace pour inhiber la croissance d'une cellule cible.

8. Utilisation d'un peptide CPF ou d'un analogue ou dérivé CPF de celui-ci suivant l'une quelconque des revendications 1 à 4, pour la fabrication d'un médicament ou d'une composition, étant un agent antitumoral.

9. Utilisation d'un peptide CPF ou d'un analogue ou dérivé CPF de celui-ci suivant l'une quelconque des revendications 1 à 4, pour la fabrication d'un médicament ou d'une composition, étant un agent antiviral.

10. Utilisation d'un peptide CPF ou d'un analogue ou dérivé de CPF de celui-ci suivant l'une quelconque des revendications 1 à 4, pour la fabrication d'un médicament, étant un agent antimicrobien.

11. Utilisation d'un peptide CPF ou d'un analogue ou dérivé CPF de celui-ci suivant l'une quelconque des revendications 1 à 4, pour la fabrication d'un médicament ou d'une composition, étant un agent antibactérien.

12. Utilisation d'un peptide CPF ou d'un analogue ou dérivé CPF de celui-ci suivant l'une quelconque des revendications 1 à 4, pour la fabrication d'un médicament ou d'une composition, étant un agent antispermicide.

13. Utilisation d'un peptide CPF ou d'un analogue ou dérivé CPF de celui-ci suivant l'une quelconque des revendications 1 à 4, pour la fabrication d'un médicament ou d'une composition, étant un agent contre les protozoaires.

14. Utilisation d'un peptide CPF ou d'un analogue ou dérivé CPF de celui-ci suivant l'une quelconque des revendications 1 à 4, pour la fabrication d'un médicament ou d'une composition, étant un antibiotique.